(19) Europäisches Patentamt
European Patent Office
Office européen des brevets

(11) EP 1 413 630 B1

(12) FASCICULE DE BREVET EUROPEEN

(45) Date de publication et mention de la délivrance du brevet:
**11.01.2012 Bulletin 2012/02**

(51) Int Cl.:
***C12Q 1/68*** *(2006.01)*

(21) Numéro de dépôt: **03104367.2**

(22) Date de dépôt: **22.02.2001**

# (54) Analyse de cibles biologiques utilisant une biopuce comportant un marqueur fluorescent

Analyse von biologischen Zielmolekülen unter Verwendung eines einen fluoreszenzmarker tragenden Biochips

Analysis of biological targets using a biochip comprising a fluorescent marker

(84) Etats contractants désignés:
**AT BE CH CY DE DK ES FI FR GB GR IE IT LI LU MC NL PT SE TR**

(30) Priorité: **23.02.2000 FR 0002236**

(43) Date de publication de la demande:
**28.04.2004 Bulletin 2004/18**

(62) Numéro(s) de document de la (des) demande(s) initiale(s) en application de l'article 76 CBE:
**01907873.2 / 1 266 030**

(73) Titulaires:
- **Commissariat à l'Énergie Atomique et aux Énergies Alternatives 75015 Paris (FR)**
- **Universite Joseph Fourier de Grenoble 38000 Grenoble (FR)**

(72) Inventeurs:
- **CUZIN, Marc 38700, CORENC (FR)**
- **PELTIE, Philippe 38760, SAINT PAUL DE VARCES (FR)**
- **FONTECAVE, Marc 38330, SAINT ISMIER (FR)**
- **DECOUT, Jean-Luc 38410, VAULNAVEYS LE HAUT (FR)**
- **DUEYMES, Cécile 12000, RODEZ (FR)**

(74) Mandataire: **Poulin, Gérard et al**
**BREVALEX**
**95, rue d'Amsterdam**
**75378 Paris Cedex 8 (FR)**

(56) Documents cités:
**WO-A-99/05320**
**WO-A-99/13105**
**WO-A-99/60158**
**US-A- 5 843 655**
**US-A- 5 861 247**
**US-A- 5 876 930**
**US-A- 5 925 525**

- **FRIER C. ET AL: "Method for preparing new flavin derivatives: Synthesis of flavin-thymine nucleotides and flavin-oligonucleotide adducts." JOURNAL OF ORGANIC CHEMISTRY, (1997) 62/11 (3520-3528)., XP002153482**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



EP 1 413 630 B1

## Description

### Domaine technique

La présente description a pour objet un support d'analyse ou « biopuce » destiné à la détermination de cibles biologiques du type ADN ou ARN.

Ce support d'analyse trouve des applications dans de nombreux domaines, en particulier en biologie pour le séquençage des génomes, la recherche des mutations, le développement de nouveaux médicaments, etc.

### État de la technique antérieure

Un support d'analyse de ce type comprend une pluralité de sondes oligonucléotidiques susceptibles de donner lieu à une hybridation avec les cibles biologiques à analyser. L'hybridation correspond à l'appariement des brins-cibles avec les brins d'ADN complémentaires disposés sur le support. Pour déterminer la nature des cibles, il est donc nécessaire de pouvoir situer quels sont les sites du support et donc les oligonucléotides qui ont donné lieu à une hybridation.

Le document Médecine/Sciences, vol. 13, n°11, 1997, pp. 1317-1324 [1] décrit des supports d'analyse de ce type.

Habituellement on détermine l'hybridation des cibles biologiques sur le support à l'aide d'un marqueur fluorescent qui est associé aux cibles biologiques, après extraction des zones intéressantes (lyse et amplification éventuelle).

Après mise en contact des cibles marquées avec le support d'analyse comportant les oligosondes, on détermine les sites où a eu lieu l'hybridation en réalisant une excitation de l'ensemble des marqueurs fluorescents, suivie d'une lecture des sites pour détecter la lumière de fluorescence réémise par les marqueurs. Les sites pour lesquels une lumière de fluorescence est détectée sont ceux qui ont fixé des molécules-cibles. Des systèmes de lecture adaptés à diverses biopuces sont décrits par exemple dans US-A-5 578 832 [2] et US-A-5 646 411 [3].

Cette technique présente l'inconvénient de nécessiter le marquage de la cible biologique avant sa mise en contact avec le support d'analyse, ce qui pose certains problèmes pratiques en ce qui concerne la quantification et/ou le rendement du marquage et le délai pour l'effectuer. De plus, le marquage à une étape donnée fige la situation et empêche de réaliser par la suite des mesures dynamiques capables de suivre une réaction d'hybridation.

On peut aussi noter que la fabrication des supports d'analyse ou « biopuces » est réalisée par différentes méthodes qui toutes posent le problème du contrôle final de la qualité des supports obtenus.

En effet, la quantité dé sondes déposées sur le support, les sites du support ou plots, ou dans des cavités prévues dans ce support, pose le problème du contrôle effectif dé la présence des oligosondes. Aucune méthode n'existe à ce jour pour quantifier et localiser avec précision les oligosondes déposées ou construites in situ sur un support d'analyse réalisé par exemple en silicium, en verre ou en matière plastique, par exemple en nylon.

La demande internationale WO 99/13105 décrit un support d'analyse comportant une pluralité d'oligonucléotides fixés sur ce support et marqués par un composé fluorescent tel que du thiazole orange. Dans ce système, l'hybridation d'un oligonucléotide complémentaire entraîne une augmentation du signal de fluorescence.

Le brevet US 5843 655 divulgue un procédé de contrôle d'un support utilisant des oligonucléotides marqués avec une sonde fluorescente afin de localiser et de déterminer la quantité d'oligonucléotides fixés sur ledit support.

### Exposé de l'invention

La présente description a pour objet précisément un support d'analyse, ou biopucé, qui permet d'assurer dans de meilleures conditions le contrôle préalable de fabrication, et de faciliter ensuite et rendre plus sensible la détermination des sites du support sur lesquels s'est produit une hybridation.

Selon la description, le support d'analyse comporte une pluralité d'oligonucléotides fixés sur ce support, et il se caractérise en ce que chacun desdits oligonucléotides est marqué par un composé fluorescent tel que la fluorescence d'un oligonucléotide marqué par ce composé est atténuée, après hybridation de cet oligonucléotide marqué avec un oligonucléotide complémentaire.

L'atténuation de fluorescence peut consister en une variation de l'intensité de fluorescence, une variation de la constante de temps de la fluorescence, ou un décalage spectral en longueur d'onde de la fluorescence.

Avec un tel support d'analyse, il n'est plus nécessaire de marquer les cibles à analyser, car la localisation des sites d'hybridation peut être détectée en vérifiant la variation de fluorescence obtenue lors de l'hybridation sur les sites d'oligonucléotides concernés.

Selon l'invention, on utilise un composé fluorescent présentant une intensité de fluorescence atténuée par l'hybridation. De préférence, cette atténuation représente 30 à 99 % de l'intensité de fluorescence avant l'hybridation

Des composés fluorescents présentant ces caractéristiques sont constitués par exemple par la flavine, la déazaflavine et leurs dérivés. De tels composés sont fixés sur des oligonucléotides par l'intermédiaire d'un bras espaceur,

par exemple de type $-(CH_2)_n-$, pour donner des conjugués flavine (déazaflavine)-oligonucléotide. Une synthèse de conjugués de ce type est décrite par C. Frier et al dans J. Org. Chem., 62, 1997, pages 3520-3528 [4].

Cette synthèse consiste à fixer un dérivé de flavine sur l'oligonucléotide par une méthode de couplage phosphoramidite ou H-phosphonate en partant d'un dérivé de formule :

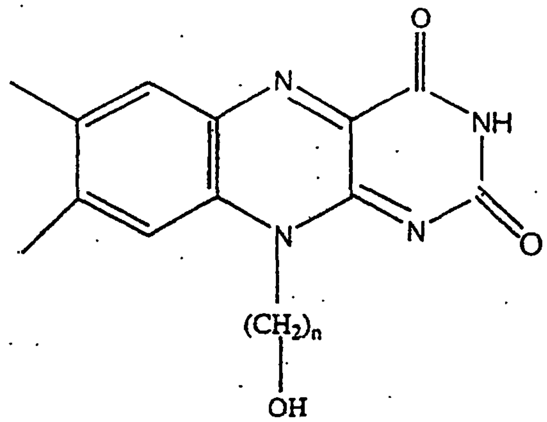

(I)

avec n étant un nombre entier de 2 à 8, par exemple 6.

La stratégie de synthèse permet d'introduire le groupe flavinique, y compris dans des procédés de synthèse oligonucléotidiques automatisés. On peut aussi utiliser cette synthèse pour la fabrication de conjugués oligonucléotides-déazaflavines.

Ainsi, l'oligonucléotide est marqué par un groupe de formule $-(CH_2)_n-R^1$ dans laquelle n est un nombre entier allant de 2 à 8, et $R^1$ représente un groupe répondant à l'une des formules suivantes

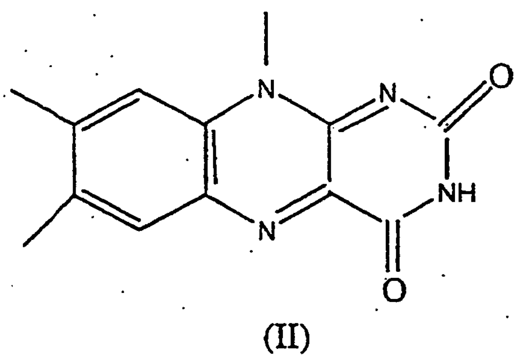

et

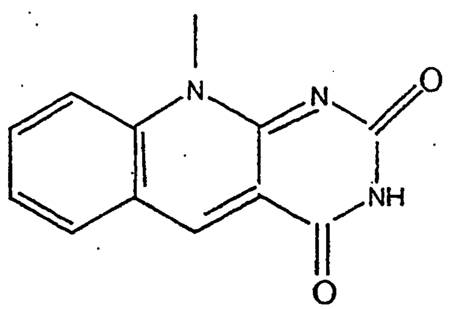

(II) (III)

L'utilisation de la flavine, de la déazaflavine ou de leurs dérivés comme composé fluorescent dans l'invention est particulièrement intéressante.

En effet, les flavines sont des molécules douées d'une intense fluorescence jaune-vert, très caractéristique. Le spectre d'excitation de fluorescence est identique au spectre d'absorption de lumière visible, avec 2 maxima à 374 et 446 nanomètres, et leur spectre d'émission de fluorescence consiste en une bande large avec un maximum vers 520 nanomètres. Les déazaflavines absorbent vers 320 et 396 nanomètres et présentent des intensités de fluorescence (émission à 452 nanomètres) plus importantes que les flavines.

Selon l'invention, on a découvert que l'accrochage de la flavine à un oligonucléotide ne modifiait pas significativement ces propriétés de fluorescence en milieu aqueux, les maxima d'excitation et d'émission ainsi que les intensités étant les mêmes pour la flavine libre et pour le conjugué oligonucléotide-flavine.

D'autre part, jusqu'à une concentration de 100 $\mu$mol.$L^{-1}$ en conjugué, il y a une excellente linéarité entre l'intensité de fluorescence et la concentration.

Il faut toutefois noter un effet de sel sur l'intensité de fluorescence, c'est-à-dire le fait que des concentrations croissantes de NaCl ont un effet croissant d'extinction « quenching » de la fluorescence des flavines. Il en est de même avec les conjugués. Ceci doit être noté car l'hybridation nécessite des concentrations en NaCl de 0,1 à 1 M pour favoriser la formation de duplex (association d'un oligonucléotide avec son oligonucléotide complémentaire).

Selon l'invention, on a découvert que lorsqu'un conjugué oligonucléotide-flavine est incubé avec un équivalent de sa cible oligonucléotidique complémentaire, il y a disparition de 90 à 100 % de la fluorescence spécifique de la flavine.

On a tiré profit de cet effet dans l'invention pour faciliter la lecture des sites d'hybridation sur des biopuces.

Aussi la description a également pour objet un procédé d'analyse de cibles biologiques par mise en contact de ces cibles avec un support d'analyse comportant une pluralité d'oligonucléotides, et détermination d'une (des) hybridation(s) entre les cibles et les oligonucléotides du support, caractérisé en ce que chacun des oligonucléotides du support est marqué par un composé fluorescent qui présente une variation de fluorescence lors de l'hybridation de l'oligonucléotide marqué avec l'oligonucléotide complémentaire, et en ce que l'on détermine les oligonucléotides du support ayant donné lieu à une hybridation par mesure de la fluorescence correspondant à chaque oligonucléotide du support avant et après la mise.en contact du support avec les cibles.

Dans ce procédé, on utilise donc le support d'analyse de la description présentant les caractéristiques décrites précédemment.

Selon une variante de ce procédé, on marque de plus les cibles par un second composé fluorescent dont la longueur d'onde d'émission est différente de celle du composé fluorescent des oligonucléotides, ou premier composé fluorescent, et l'on contrôle les oligonucléotides ayant donné lieu à une variation de fluorescence par mesure de la fluorescence de ce second composé.

Dans ce cas, le composé fluorescent des oligonucléotides du support, ou premier composé fluorescent, est choisi parmi la flavine, la déazaflavine et leurs dérivés, et le second composé fluorescent est choisi parmi des fluorophores stables utilisés sur des puces à ADN et émettant préférentiellement dans le visible (Rhodamine, $CY_3$, $CY_5$ etc.), par exemple décalés vers le rouge par rapport à la flavine.

Avec le support d'analyse de la description dont les oligonucléotides sont marqués par un composé fluorescent, on peut de plus opérer un contrôle de la qualité du support.

Aussi l'invention a pour objet un procédé de contrôle d'un support d'analyse comportant une pluralité d'oligonucléotides fixés sur ce support, caractérisé en ce que les oligonucléotides fixés sont des oligonucléotides marqués par un composé fluorescent et en ce que l'on localise et détermine la quantité d'oligonucléotides fixés sur le support par mesure de la fluorescence du composé fluorescent, chacun desdits oligonucléotiques étant marqué par un groupe de formule $-(CH_2)_m-R^1$ telle que précédemment définie.

D'autres caractéristiques et avantages de l'invention apparaîtront mieux à la lecture de la description qui suit, donnée bien entendu à titre illustratif et non limitatif, en référence aux dessins annexés.

## Brève description des dessins

La figure 1 est une courbe illustrant l'évolution de l'intensité de fluorescence du conjugué $T_{11}$-déazaflavine en fonction du nombre d'équivalents de la cible poly A (SEQ ID NO : 1).

La figure 2 est une courbe illustrant l'évolution de l'intensité de fluorescence du conjugué $T_{11}$-flavine en fonction du nombre d'équivalents de cibles poly A (SEQ ID NO : 1).

La figure 3 représente les spectres de fluorescence obtenus par hybridation du conjugué SEQ ID NO : 12-flavine avec l'oligonucléotide complémentaire (SEQ ID NO : 13) pour des additions croissantes d'équivalents de la séquence complémentaire.

La figure 4 illustré l'évolution du rapport d'intensités de fluorescence $I_0/I$ en fonction de la concentration [Q] en composé fluorescent dans le cas d'une extinction dynamique.

La figure 5, représente l'évolution du rapport d'intensités de fluorescence $I_0/I$ en fonction de la concentration [Q] en composé fluorescent dans le cas d'une extinction statique.

La figure 6 illustre l'évolution du rapport $I_0/I$ en fonction de la concentration [Q] en composé fluorescent dans le cas d'une action combinée d'extinction statique et dynamique.

La figure 7 est une courbe illustrant l'évolution $I_0/I$ en fonction de la concentration [Q] en flavine dans le cas des spectres représentés sur la figure 3.

La figure 8 est une courbe représentant l'évolution de la constante apparente $K_{app}$ en fonction de la concentration [Q] en flavine dans le cas des spectres de la figure 3.

## Exposé détaillé des modes de réalisation

Dans les exemples qui suivent, on utilise les oligonucléotides suivants

Cible $T_{11}$ (SEQ ID NO : 1) :
5' ctcatcgtgt aaaaaaaaaa aggcagtact ggaagggcta attct 3'.

Cible $T_{16}$ (SEQ ID NO : 2) :
5'ctcatcgtga attctaaaaa aaaaaaaaaa agctaacagc agtac 3'

Oligonucléotide complémentaire de SEQ ID NO : 1. (SEQ ID NO : 3) :
3' gagtagcaca tttttttttt tccgtcatga ccttcccgat taaga 5'

Oligonucléotide complémentaire de SEQ ID NO : 2. (SEQ ID NO : 4).
3' gagtagcact taagattttt tttttttttt tcgatcgccg tcatg 5'

oligonucléotide HIV (SEQ ID NO : 5) :
5' -ttttc$^{me}$ttttg gggggt-3'

Double brin de cible HIV (SEQ ID NO : 6 et 7).
5' cagtcccccc ttttctttta aaaagtggct aagatctac 3' (SEQ ID NO : 6)
3' gtcagggggg aaaagaaaat ttttcaccga ttctagatg 5' (SEQ ID NO : 7)

**Exemple 1 :**

Dans cet exemple, on prépare les conjugués flavine-oligonucléotide $T_{11}$ et flavine-oligonuclétide $T_{16}$ en suivant le mode opératoire décrit dans le document [4] en utilisant le dérivé de flavine F1 de formule (I) avec n = 6. On obtient ainsi le conjugué (SEQ ID NO : 8) de formule :

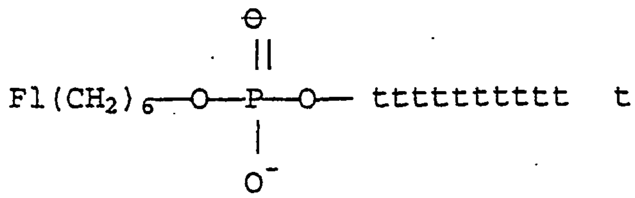

On prépare de la même façon un conjugué flavine-$T_{16}$ (SEQ ID NO : 9) de formule :

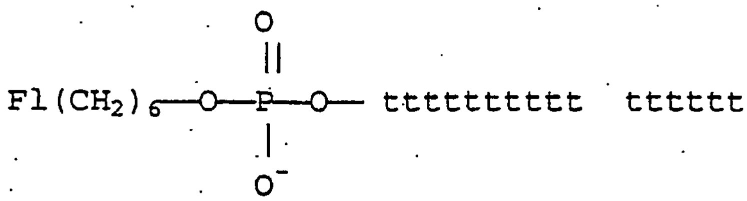

On prépare enfin le conjugué HIV-flavine en utilisant le dérivé de flavine de formule (I) avec n = 6. (SEQ ID NO : 10) de formule :

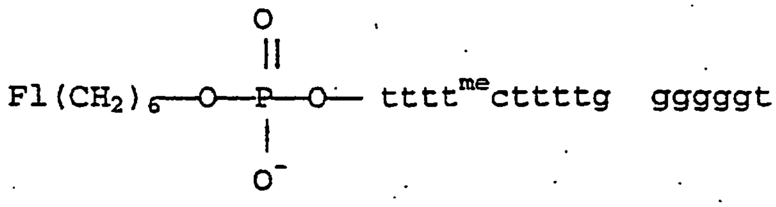

On prépare de la même façon le conjugué $T_{11}$-déazaflavine DF1 (SEQ ID NO : 11) de formule :

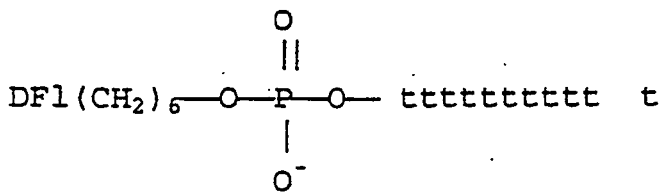

**Exemple 2 :**

Dans cet exemple, on étudie l'influence de la présence d'un autre oligonucléotide sur l'émission de fluorescence du conjugué $T_{11}$-flavine (SEQ ID NO : 8).

Dans ce but, on met en présence 0,1 à 10 $\mu$mol.$L^{-1}$ du conjugué $T_{11}$-flavine avec 1 équivalent de l'oligonucléotide (SEQ ID NO : 1), dans un tampon Tris-HCl 10 mM, pH 7,0 avec 0,1 mM-1MNaCl.

On réalise l'excitation à 460 nanomètres et on mesure l'émission de fluorescence autour de 540 nanomètres.

Dans ces conditions, l'émission de fluorescence ne représente plus que 5 % de l'émission mesurée sur le conjugué $T_{11}$-flavine seul.

On recommence cette opération en présence' de l'oligonucléotide (SEQ ID NO : 3), c'est-à-dire l'oligonucléotide complémentaire de SEQ ID NO : 1. Dans ces conditions, l'émission de fluorescence représente 98 % de l'émission de fluorescence du conjugué $T_{11}$-flavine. La présence de cet oligonucléotide n'a donc pas eu d'influence sur l'émission de fluorescence du conjugué $T_{11}$-flavine.

Si l'on recommence cette opération en ajoutant 1 équivalent de l'oligonucléotide SEQ ID NO : 1 et 1 équivalent de l'oligonucléotide SEQ ID NO : 3, dans les conditions où le duplex ne peut pas se former, l'émission de fluorescence représente 6 % de l'émission de fluorescence du conjugué seul.

Ainsi, il est clair que la présence de la cible complémentaire (SEQ ID NO : 1), de l'oligonucléotide $T_{11}$ conduit à une disparition de 90 à 100 % de la fluorescence spécifique de la flavine.

**Exemple 3 :**

On effectue les mêmes expériences sur le conjugué T16-flavine (SEQ ID NO : 9), soit en présence de la cible $T_{16}$ (SEQ ID NO : 2), soit en présence de l'oligonucléotide (SEQ ID NO : 4), complémentaire de (SEQ ID NO : 2), soit en présence du mélange des deux oligonucléotides SEQ ID NO : 2 et SEQ ID NO : 4, dans les conditions où le duplex ne peut pas se former.

On obtient les résultats suivants

| **Oligonucléotides** | **Emission à 540 nm** |
|---|---|
| $T_{16}$-flavine (SEQ ID NO : 9),<br>avec sa cible (SEQ ID NO : 2), | 100 %<br>2 % |
| avec l'oligonucléotide (SEQ ID NO : 4), complémentaire de la cible, | 91 % |
| avec SEQ ID NO : 2 et SEQ ID NO : 4, (conditions où le duplex ne se forme pas) | 4% |

Ainsi, comme dans le cas de l'exemple 2, la présence de la cible (SEQ ID NO : 2) de l'oligonucléotide $T_{16}$

conduit à une extinction de fluorescence de la flavine en raison de l'hybridation entre la.cible et l'oligonucléotide $T_{16}$.

**Exemple 4 :**

Dans cet exemple, on teste les propriétés de l'oligonucléotides HIV conjugué à la flavine (SEQ ID NO : 10), en présence du duplex HIV formé par les SEQ ID NO : 6 et SEQ ID NO : 7.

Dans ces conditions, l'émission de fluorescence représente 90 % de l'émission initiale. Il n'y a pas eu d'hybridation entre le conjugué F1-HIV et le duplex puisque la cible de HIV avait déjà donné lieu à une hybridation.

Les exemples 2 à 4 montrent ainsi que l'extinction de fluorescence est spécifique de la formation d'un duplex entre le conjugué oligonucléotide-flavine et l'oligonucléotide complémentaire pour les raisons suivantes :

1) Une élévation de température, qui conduit à une déstabilisation du duplex et à un déplacement de l'équilibre : double-brin→simples brins fait remonter l'intensité de fluorescence. Il est à noter que la spectroscopie de fluorescence constitue donc une bonne méthode pour évaluer les constantes d'association à une température donnée. Une nouvelle baisse de température diminue à nouveau l'intensité de fluorescence.
2) L'addition d'un oligonucléotide non complémentaire du conjugué ne conduit pas à une diminution de la fluorescence.
3) L'addition d'un double brin, complémentaire du conjugué à une solution de conjugué oligonucléotide-flavine dans des conditions .qui conduisent à la formation de triplex ne conduit pas à une extinction de fluorescence, comme il apparaît dans l'exemple 4.
4) Lorsque la cible est un ARN complémentaire, on observe également une diminution de la fluorescence mais à un degré moindre.

On peut encore noter que le doublet gg adjacent à la séquence $A_{11}$ de l'oligonucléotide SEQ ID NO : 1, qui ne participe pas à l'appariement, n'est pas nécessaire puisque des résultats identiques ont été obtenus avec des oligonucléotides identiques à SEQ ID NO : 1, mais comportant à la place de gg, les séquences gc, cg et cc.

**Exemple 5 :**

Dans cet exemple, on étudie l'influence de la présence de l'oligonucléotide cible SEQ ID NO : 1 à des concentrations allant jusqu'à 1,5 équivalent pour 1 équivalent du conjugué $T_{11}$-déazaflavine (SEQ ID NO : 11) sur la fluorescence de la déazaflavine du conjugué. On opère dans les mêmes conditions que celles des exemples 2, 3 et 4

La figure 1 illustre les résultats obtenus, soit l'évolution de l'intensité de fluorescence à 452 nanomètres en fonction du nombre d'équivalents de la cible poly A (SEQ ID NO : 1).

**Exemple 6 :**

Dans cet exemple, on effectue la même expérience que dans l'exemple 5, mais en utilisant comme conjugué le conjugué $T_{11}$-flavine (SEQ ID NO : 8).

La figure 2 illustre les résultats obtenus, soit l'intensité de fluorescence à 520 nanomètres en fonction du nombre d'équivalents de la cible poly A (SEQ ID NO : 1).

Une comparaison des figures 1 et 2 montre que les écarts de fluorescence sont plus importantes avec la déazaflavine qui est de ce fait plus sensible.

**Exemple 7 :**

Dans cet exemple, on étudie la stabilité du complexe $T_{11}$-flavine (SEQ ID NO : 8) avec la SEQ ID NO : 1 complémentaire. On effectue des expériences de gel-retard et de mesure de la température de fusion Tm. On a ainsi pu montrer que la présence d'une flavine ou d'une déazaflavine en bout d'un oligonucléotide confère un important surcroît de stabilité au complexe que fait cet oligonucléotide avec l'oligonucléotide complémentaire.

Par exemple, à une concentration en NaCl de 0,1 M, la séquence $T_{11}$ conduit à un complexe avec l'oligonucléotide SEQ ID NO : 1 avec un Tm de 23°C (37°C à 1 M NaCl), tandis que le conjugué $T_{11}$-flavine (SEQ ID NO : 8) donne avec SEQ ID NO : 1, un complexe avec un Tm de 28°C (42°C à 0,1 M NaCl).

Des expériences de gel-retard montrent que la formation du duplex nécessite des quantités beaucoup moins grandes de conjugué $T_{11}$-flavine (SEQ ID NO : 8) que d'oligonucléotide $T_{11}$ sans flavine.

On a trouvé des résultats similaires avec le conjugué $T_{16}$-flavine (SEQ ID NO : 9) et la cible (SEQ ID NO : 2) par rapport à $T_{16}$ sans flavine.

L'étude des congugués d'oligonucléotides- flavines (SEQ ID NO : 8 et 9) révèle une propriété tout à fait intéressante de ces molécules :

- la présence de la flavine stabilise l'association de ce conjugué avec l'oligonucléotide complémentaire, par un mécanisme encore mal compris (liaisons hydrogène entre la flavine et les bases nucléiques, stacking, ...)
- cette association conduit à une diminution très importante de la fluorescence (extinction dans certains cas),
- cette extinction de fluorescence est très spécifique : elle ne se produit que lorsque le conjugué se trouve en présence de l'oligonucléotide complémentaire, dans des conditions de formation du duplex.

**Exemple 8 :**

Dans cet exemple, on met en contact un conjugué formé d'un oligonucléotide de 20 méres et de flavine (SEQ ID NO : 12) :

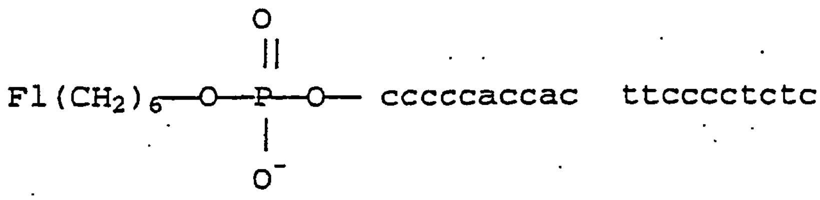

avec l'oligonucléotide complémentaire (SEQ ID NO : 13) :
ctagcctgat gaaaggggaa gtggtggggg agacatagcc
en concentration croissante, dans les mêmes conditions que celles des exemples 2 à 4, et on mesure le spectre de fluorescence autour de 520 nanomètres au spectrofluorimètre.

La longueur d'onde d'excitation est de 446 nanomètres et l'émission maximale correspondant à la flavine est à 520 nanomètres.

On effectue la mesure instantanément, puis 5 minutes après, avec à chaque fois une concentration plus importante de la cible complémentaire (SEQ ID NO : 13).

La figure 3 illustre les spectres de fluorescence obtenus sans l'oligonucléotide complémentaire, puis avec 0,2, 0,4, 0,6, 0,8, 1, 1,2 et 1,4 équivalents de l'oligonucléotide complémentaire SEQ ID NO : 13.

A partir d'une intensité initiale de 318 en l'absence de la cible (l'oligonucléotide complémentaire), on observe une extinction de fluorescence d'un rapport 20 environ lorsque que l'on met en léger excès la cible complémentaire.

L'extinction peut correspondre à divers processus qui peuvent être soit un transfert d'énergie, soit une extinction par collision (« quenching collisionnel »), soit la formation de complexe (« quenching statique ») qui correspond à l'hybridation sonde-cible.

L'extinction par collision ou (« quenching collisionnel » intervient lorsqu'il y a collision entre le composé fluorescent (flavine) et l'oligonucléotide extincteur en concentration [Q]. Il est décrit par la loi de Stern-Volmer qui correspond à l'équation suivante :

$$I_0/I = 1 + h_q \tau_0 [Q]$$

où $h_q$ représente la constante d'extinction et $\tau_0$ est la durée de vie de fluorescence.

La constante de Stern-Volmer est définie de la façon suivante :

$$K_D = h_q \tau_0 .$$

Dans ce cas, d'une part, la durée de vie devient $\tau$ et $I_0/I = \tau_0/\tau$, d'autre part la température a pour effet d'accroître le phénomène, Kd augmente lorsque la température augmente.

La figure 4 illustre l'évolution de $I_0/I$ et de $\tau 0/\tau$, en fonction de [Q]. Lorsqu'on augmente la température, la pente de.la droite augmente.

Dans le cas d'une extinction par formation de complexe, c'est-à-dire dans le cas qui nous intéresse, l'hybridation sonde/cible, soit le « quenching statique », la loi de Stern-Volmer devient :

$$I_0/I = 1 + K_s [Q]$$

où $K_s$ est la constante d'association.

La figure 5 illustre l'évolution de $I_0/I$ en fonction de [Q]. Dans ce cas, $\tau0/\tau = 1$ et la pente diminue lorsque la température augmente, ce qui est normal puisqu'un accroissement de température provoque une dénaturation partielle du complexe, donc le fluorophore qui était éteint se trouve libéré.

Dans la plupart des cas, comme représenté sur la figure 6, on trouve conjointement une action combinée de « quenching » statique et dynamique, ce qui se traduit par une courbure positive vers l'axe y. La relation de Stern-Volmer doit être ainsi modifiée

$$I_0/I = 1 + (K_d + K_s) [Q] + K_d K_s [Q]^2$$

Si l'on revient aux spectres de fluorescence illustrés sur la figure 3, et que l'on trace la loi $I_0/I$ en fonction de [Q] à partir des spectres, on trouve de fait une courbure positive et non une droite, ce qui traduit la présence simultanée de « quenching » statique et dynamique, comme il apparaît sur la figure 7.

La figure 8 illustre la variation de $K_{app}$ en fonction de la concentration [Q] en équivalents à partir des résultats de la figure 3.

On accroche à la cible un deuxième fluorophore, non sujet au « quenching » et fortement décalé en longueur d'onde de fluorescence par rapport au composé fluorescent. Ainsi, ce fluorophore pourra être excité parallèllement et révèlera la présence effective du complexe d'hybridation.

On peut utiliser en particulier un fluorochrome tel que $CY_3$ qui a une longueur d'onde d'excitation égale à 543,5 nanomètres et une longueur d'onde d'émission égale. à 580 nanomètres. Il peut être révélé avec un scanner utilisant un laser HeNe vert de faible puissance (1 mW) tel qu'il est fait dans un système de lecture de biopuces.

Ce double marquage, c'est-à-dire celui de l'oligonucléotide fixé sur la biopuce au moyen d'un premier composé fluorescent tel que la flavine, et celui des cibles à déterminer par un second composé fluorescent tels que $CY_3$, possède des avantages indéniables par rapport à un simple marquage traditionnel.

En effet, lors de l'immobilisation des sondes sur la biopuce, la présence de flavine permet un contrôle de la qualité des sondes avant hybridation, en faisant une image totale de la puce de sorte que tous les sites possédant des sondes fluorescent. Il y a alors possibilité de cartographier les défauts de la puce, (image non homogène en intensité de fluorescence) ; il y a aussi possibilité de profiter de la redondance en mettant le même type de sonde sur plusieurs sites contigus.

Lors de la phase d'hybridation, on observe une diminution de fluorescence sur les sites concernés avec une lecture à 520 nanomètres en excitant autour de 400-450 nanomètres. Une lecture de $CY_3$ permet alors de résoudre la non-spécificité puisque seuls les complexes hybridés posséderont la cible $CY_3$ conjointement à la décroissance de fluorescence.

L'intérêt de plages de longueurs d'onde très distinctes réside dans le fait que l'excitaUon à 400-450 nanomètres ne fait quasiment pas fluorescer $CY_3$ car on est très loin de sa bande d'absorption (absorption maximale à 550 nanomètres).

Un paramètre intéressant qui peut être évalué est la durée de vie qui, on l'a vu, ne varie pas lors de la formation de complexes mais varie fortement dans le cas de « quenching » dynamique. On peut donc facilement séparer les deux phénomènes en compétition : le « quenching » collisionnel et la formation d'hybrides qui seule est intéressante

Un autre paramètre qu'il convient de suivre est l'évolution de la fluorescence en fonction de la température. En effet, dans une "puce" traditionnelle, on lit la fluorescence à une température donnée. L'élévation de température a pour effet de "casser" les hybridations non-spécifiques (dénaturation.) mais a aussi pour effet fâcheux de réduire la fluorescence du complexe (puisque le rendement quantique de fluorescence diminue lorsque la température augmente). Dans le cas de « quenching » statique, on a vu que l'élévation de température restaure au contraire la fluorescence des complexes mal hybridés puisqu'il y a "libération" du fluorophore incriminé.

Ainsi, l'association judicieuse d'une lecture à 2 marqueurs (flavine + fluorophore traditionnel), contrôle en température, voire mesure de durée de vie, permet de suivre parfaitement la formation des hybrides et même de calculer les différentes constantes (constante de Stern-Volmer, constante d'association, voire même calcul des rayons des sphères d'action).

**Références citées**

[1] : Médecine/Sciences, vol. 13, n°11, 1997, pp. 1317-1324
[2] : US-A-5 578 832
[3] : US-A-5 646 411
[4] : C. Frier et al, J. Org. Chem., 62, 1997, pages 3520-3528.

## Revendications

1. Procédé de contrôle d'un support d'analyse comportant une pluralité d'oligonucléotides fixés sur ce support, **caractérisé en ce que** les oligonucléotides fixés sont des oligonucléotides marqués par un composé fluorescent et **en ce que** l'on localise et détermine la quantité d'oligonucléotides fixés sur le support par mesure de la fluorescence du composé fluorescent, avant et après hybridation avec des oligonucléotides complémentaires,
chacun desdits oligonucléotides étant marqué par un groupe de formule $-(CH_2)_n-R^1$ dans laquelle n est un nombre entier allant de 2 à 8 et $R^1$ représente un groupe répondant à l'une des formules suivantes :

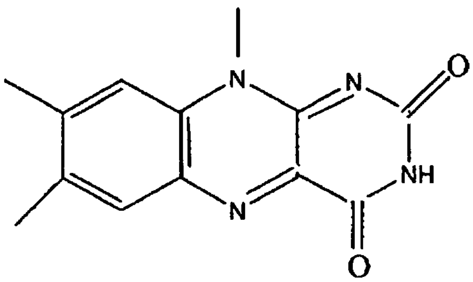

(II)

et

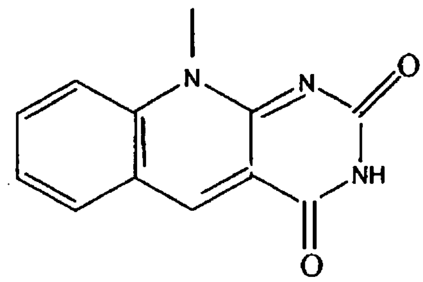

(III)

2. Procédé selon la revendication 1, dans lequel n est 6.

## Claims

1. Control method for an analytical support comprising a plurality of oligonucleotides fixed on that support, **characterized in that** the fixed oligonucleotides are oligonucleotides marked by a fluorescent compound and **in that** the quantity of oligonucleotides fixed on the support is localized and determined by measurement of the fluorescence of the fluorescent compound, before and after hybridization with complementary oligonucleotides,
each of said oligonucleotides being marked by a group of formula $-(CH_2)_n-R^1$ in which n is an integer from 2 to 8 and $R^1$ represents a group corresponding to one of the following formulas :

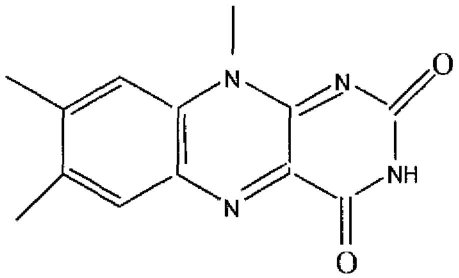

(II)

and

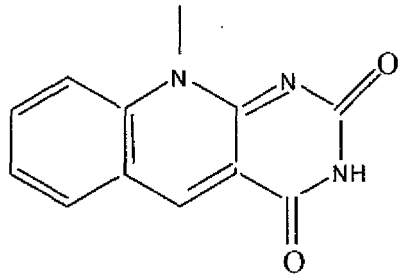

(III)

2. Method according to claim 1, in which n is 6.

**Patentansprüche**

1. Verfahren zur Prüfung eines Analysenträgers, der eine Mehrzahl auf diesem Träger fixierter Oligonukleotide umfasst, **dadurch gekennzeichnet, dass** die fixierten Oligonukleotide durch eine fluoreszierende Verbindung markierte Oligonukleotide sind und **dadurch**, dass die Menge der auf dem Träger fixierten Oligonukleotide durch Messung der Fluoreszenz der fluoreszierenden Verbindung vor und nach der Hybridisierung mit komplementären Oligonukleotiden lokalisiert und bestimmt wird, wobei jedes dieser Oligonukleotide durch eine Gruppe Formel $-(CH_2)_n-R^1$ markiert ist, worin n eine ganze Zahl von 2 bis 8 ist und $R^1$ eine einer der folgenden Formeln entsprechende Gruppe ist:

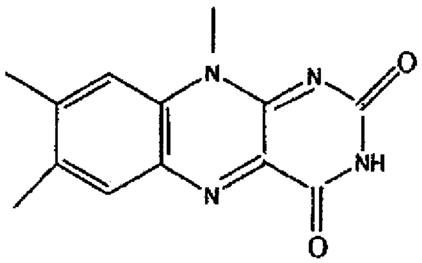

(II)

und

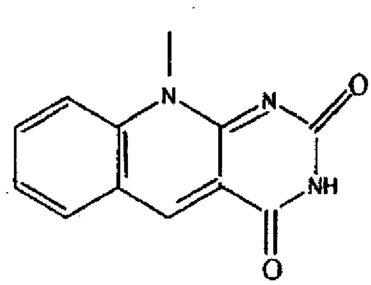

(III)

2. Verfahren gemäß Anspruch 1, wobei n 6 ist.

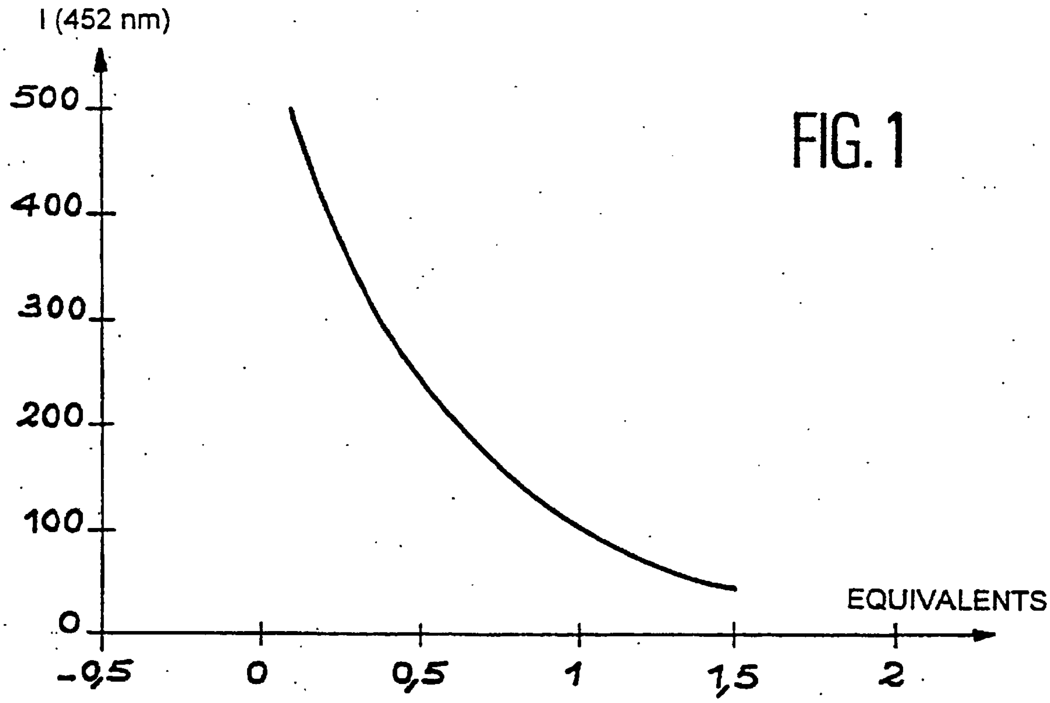
I (452 nm)
500
400
300
200
100
0
EQUIVALENTS
-0,5
0
0,5
1
1,5
2

FIG. 1

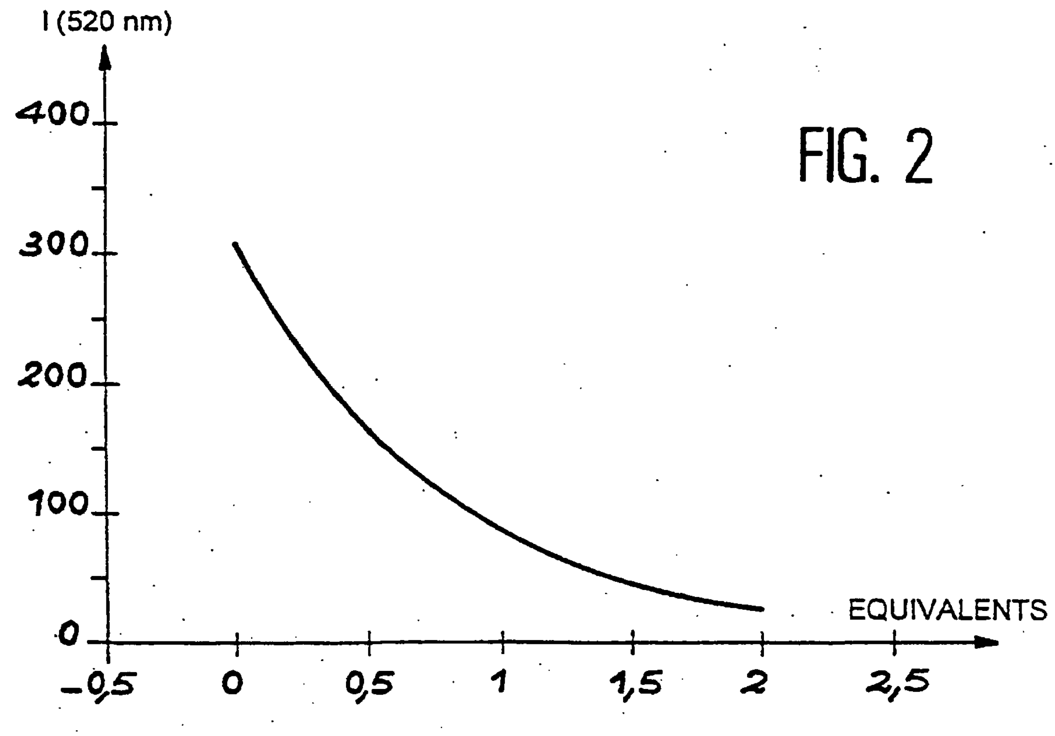
I (520 nm)
400
300
200
100
0
EQUIVALENTS
-0,5
0
0,5
1
1,5
2
2,5

FIG. 2

FIG. 3

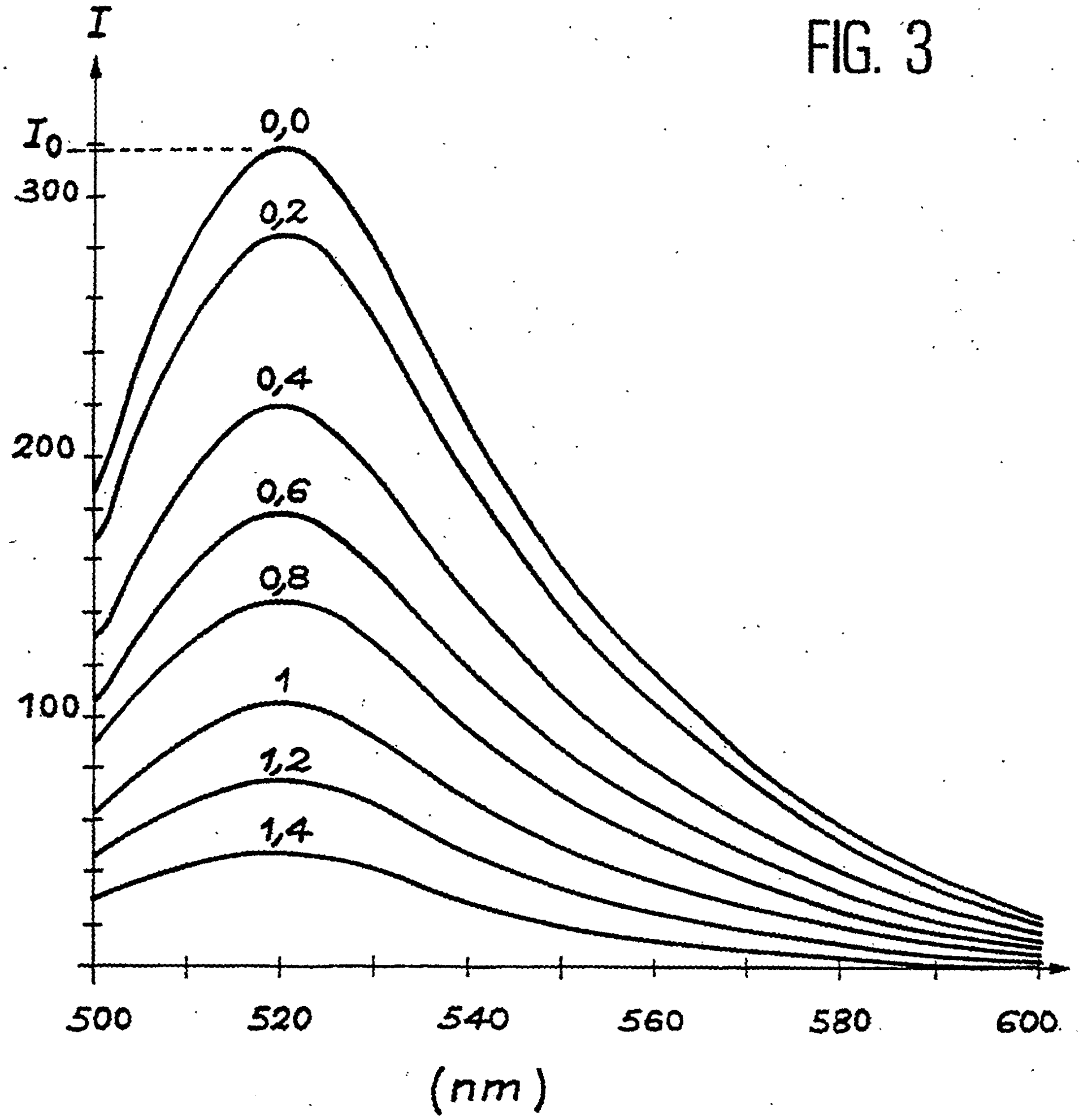

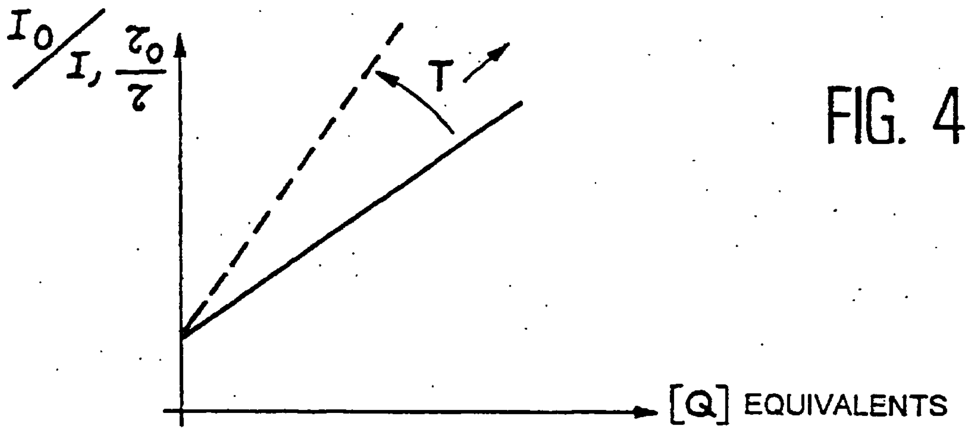


FIG. 4

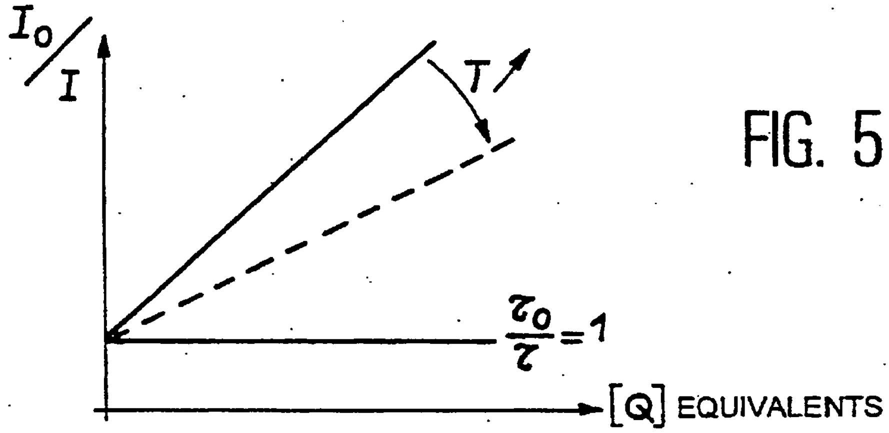


FIG. 5

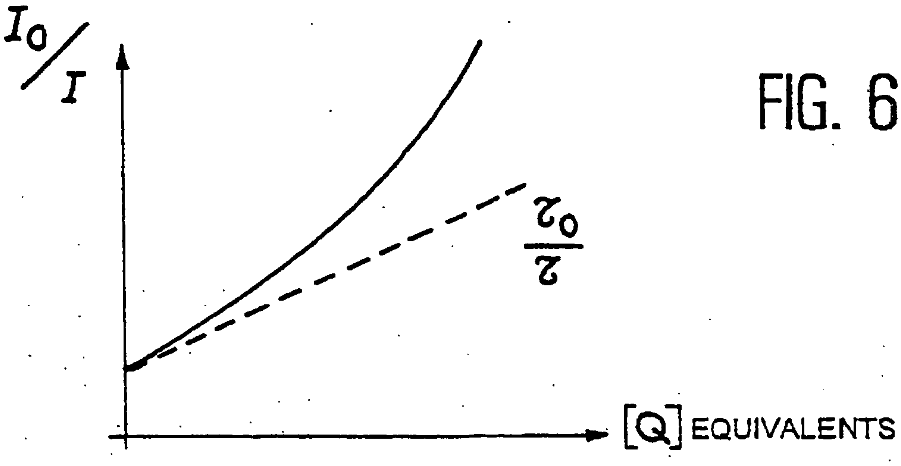


FIG. 6

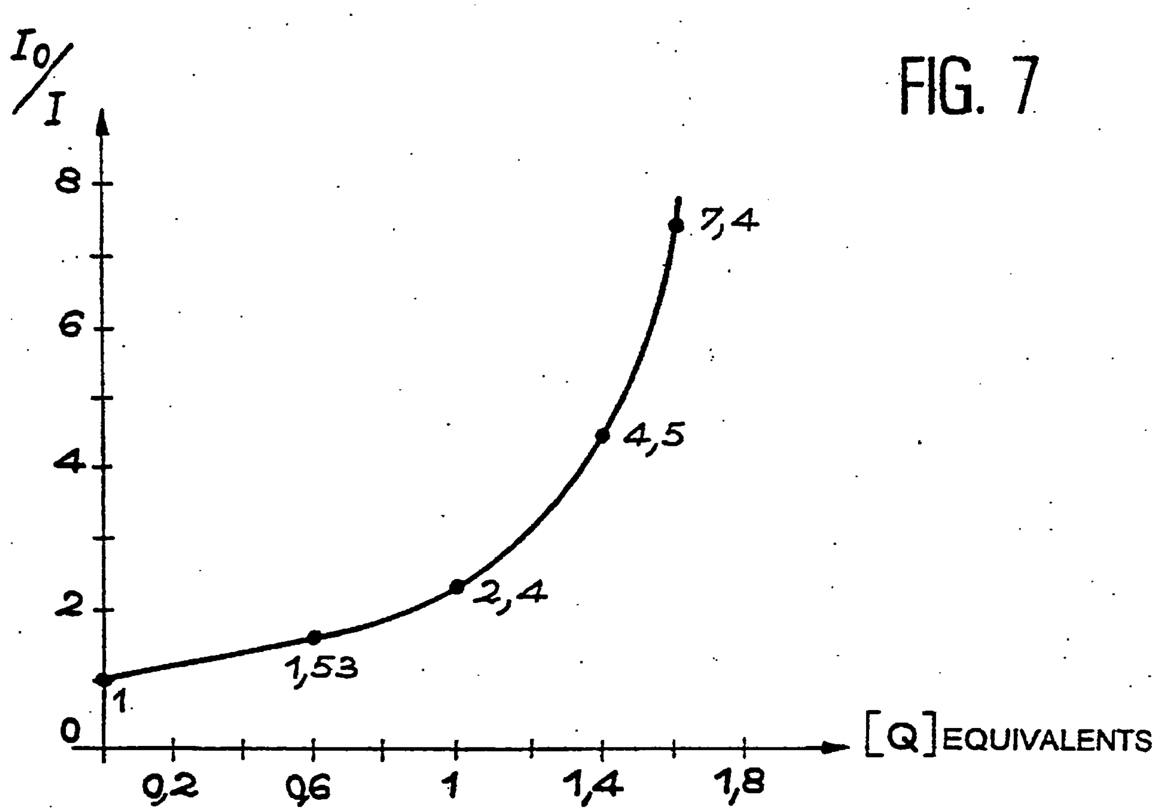

I0/I
8
6
4
2
0
7,4
4,5
2,4
1,53
1
0,2
0,6
1
1,4
1,8
[Q] EQUIVALENTS


FIG. 7

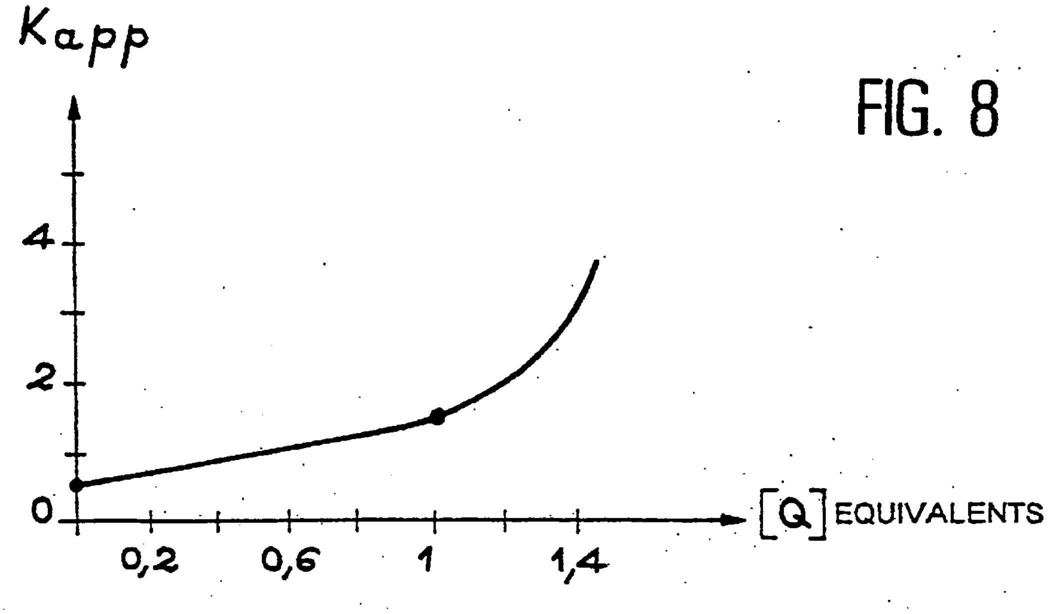

Kapp
4
2
0
0,2
0,6
1
1,4
[Q] EQUIVALENTS


FIG. 8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 5578832 A
- US 5646411 A
- WO 9913105 A
- US 5843655 A

### Littérature non-brevet citée dans la description

- *Médecine/Sciences,* 1997, vol. 13 (11), 1317-1324
- **C. Frier et al.** *J. Org. Chem.,* 1997, vol. 62, 3520-3528